# EUROPEAN PATENT SPECIFICATION

(11) **EP 1 021 137 B1**
(45) Date of publication and mention of the grant of the patent: **08.08.2007**
(21) Application number: 98946740.2
(22) Date of filing: 06.10.1998
(51) Int. Cl.: A61D 7/00, A61F 6/14, A61M 31/00, A61K 9/00

(54) **DRUG DELIVERY SYSTEM**
ANORDNUNG ZUR MEDIKAMENTENZUFÜHRUNG
SYSTEME D'ADMINISTRATION DE MEDICAMENTS

(30) Priority: 10.10.1997 NZ 32896797
(43) Date of publication of application: 26.07.2000
(73) Proprietor: Bioniche Life Sciences Inc., Belleville, Ontario K8N 1E2 (CA)
(72) Inventor: DUIRS, Graham, Francois, Hamilton 2001 (NZ)
(74) Representative: Cummings, Sean Patrick
(86) International application number: PCT/NZ1998/000147
(87) International publication number: WO 1999/018884

(56) References cited:
- EP-A2- 0 715 847
- WO-A1-88/04544
- WO-A1-93/19698
- WO-A1-96/01092
- AU-A- 7 607 474
- DE-A- 3 332 156
- US-A- 3 788 296
- US-A- 4 341 728
- US-A- 4 369 783

## Description

### TECHNICAL FIELD

This invention relates to a substance delivery system.

Reference throughout the specification shall be made to the use of the present invention as a drug delivery system for use in animal body cavities, such as the vagina.

It should be appreciated however that the present invention can be used to deliver substances other than drugs and can be used in relation to humans and in other body cavities, for example the rumen, ears, mouth and so forth.

Drug delivery systems are used extensively in controlled breeding and reproductive management. Although considerable research has been invested in the design of these devices, there are still problems associated with them.

Firstly, these devices are required to be retained within the body cavity for the slow release of drugs over a period of time. To facilitate this, various arms and projections have been built into the device which can either engage with the walls of the body cavity, or make the device wide enough such that when in the body cavity the device cannot naturally exit the animal through the entrance orifice.

Major problems with the provision of such arms or projections is that they can irritate or even rupture the lining of the body cavity, causing distress to the animal and providing a site for possible infection. Yet another problem with these projections is that in order for the device to be inserted into the animal, the device will need to be considerably smaller than it is when the projections are fully extended. Thus, the device needs to be designed so the projections can be retracted or folded away during insertion and removal of the device.

A major problem with drug delivery devices is that traditionally they have been manufactured with the drug impregnated into the material from which the device is made. Typically, this material is in many instances a matrix of silicone.

To manufacture devices from drug impregnated silicone is expensive.

A further disadvantage of using a drug impregnated device is that it is very difficult to dispose. For example, the hormones used in reproductive management are required to be disposed in accordance with heavily regulated environmental procedures. As it is always possible that the drug within the silicone matrix had not been fully delivered to the animal when the device is removed, the whole device will have to be disposed as the whole device is the drug delivery system.

It would be desirable if the devices could be reused.

Another problem with the devices is that they have a specific dose rate which cannot be readily changed. Further with these devices, the treatment cannot be changed or customised according to requirements. For instance, animals at the heavier end of the species weight range may require a dose supplement or a type of breed may vary in size and require a dose change.

An example of a substance delivery system in described in WO-A1-9601092 in the form of an intra-uterine device comprising an elongated stem joined to an elongated transverse part that comprises a polymer matrix reservoir containing an active agent.

It would be desirable if there could be provided a drug delivery device for use inside body cavities which was easy to insert, readily retained within the cavity without irritation to the cavity walls, was reusable, could allow for differing treatments and was comparatively inexpensive.

### BACKGROUND ART

it is an object of the present invention to address the foregoing problems or at least to provide the public with a useful choice.

Further aspects and advantages of the present invention will become apparent from the ensuing description which is given by way of example only.

### DISCLOSURE OF THE INVENTION

According to the present invention there is provided an internal substance delivery device as defined in claim 1.

The substance delivery device should now be referred to as a drug delivery device such as an intravaginal release device.

It should be appreciated however that a device in accordance with the present invention can be adapted for use in other body cavities, such as the rumen, the auditory system and so forth. It should also be appreciated that the present invention can be used in both humans and animals.

Further, it should be appreciated that the substance being delivered can be in a variety of forms, e.g. liquid, solid bullets, powder, gel and so forth.

The support frame may come in a number of configurations. The main purpose of the support frame is to hold the substance delivery pods in such a manner that they can deliver the substance effectively to the body cavity.

Other requirements of the support frame is that it is naturally retained within the body cavity when required for the delivery of substance, but can also be readily inserted and removed.

The applicant has designed support frames with a number of configurations which meet the above criteria. One particular set of designs the applicant has arrived at has at least two arms which are pliable in movement situations, that maintains a tension across the length of the arms.

However, reference throughout this specification will now be made to the drug delivery device as having removable substance delivery means in the form of pods.

It should be appreciated that the prior art devices were fairly inflexible having straight arms rigidly fixed to the main body of this device when *in situ.* In contrast, the applicant has found that curved arms give considerable pliability and/or tension.

For ease of reference, throughout the specification the support frames shall be referred to as having two arms.

It should be appreciated however that the present invention can have any number of arms and that two arms is merely just one form of a preferred embodiment.

The applicant believes that having arms which are pliable in movement situations means that the device is less harsh on the interface with the mucosal membrane in the vagina. For example, animal movement or change of position will enable the device to flex accordingly to facilitate animal comfort and yet maintain retention integrity characteristics.

One way by which such a design can be achieved is to create a "wishbone" shape. That is, a comparatively short joining piece or base and two arms which curve firstly outwards from the base and then inwards to provide a substantially S-shaped arms.

If the arms curve away from the base so as to form the outline of the bowl, there is no tension where the arms connect to the base. However, there is tension throughout the arms provided by the double curve of the S-shape.

The lack of tension at the base means that the arms can still move with respect to the base if required. However the tension along the length of the arms can cause the arms to bias outwards from the body of the support frame causing the arms or the substance delivery pods attached to arms to extend outwards toward the mucosal membrane, and in some cases exposing the surface of the pods to the mucosal membrane.

It should be noted to here that the mucosal membrane is very effective at transferring drugs to the body. And, while it is not a necessity, it can be beneficial to drug delivery.

It should be appreciated that other configurations are envisaged. For example, one embodiment present invention may be in the shape of a part circle, such as a "bicycle clip" configuration which requires no base: but still has the tension and pliability in the arms. Other embodiments may have the arms, not curving away from the base in a bowl shape, but curving in the opposite direction.

It should be appreciated that the base can be used to locate and remove the device.

There is now greater choice in the material from which the support frame can be made. This is because the present invention obviates the need to impregnate the support frame with the substance to be delivered. This is because the drug delivery pods are attachable and removable from the support frame. Thus, manufacture of the support frame is quite independent of the drug delivery system.

In further embodiments however the support frame is made of a plastics material such as nylon which is readily moulded, flexible and is physiologically friendly and reusable. Other materials may of course be used.

The term pod should not be seen as limiting as is intended to mean any article which can be attached to or detached from the support frame and capable of releasing substances such as drugs.

However, in preferred embodiments of the present invention the pods are devices which house or incorporate the substance to be delivered.

The pods may release the substances into the body cavity by a variety of means. In one embodiment, this may be through a simple process of osmosis of the drug passing through a membrane on the pod.

In other embodiments it may be a device in the pod which applies pressure to the drug pushing it out of the pod for instance, through micropores.

In other embodiments there may be electronically controlled release of the substance.

The pods can take any suitable shape. However, it is preferable that the pods do not have projections which could irritate the lining of the body cavity. Instead, it is envisaged that the outer surfaces of the pods are smooth and possibly rounded. In one embodiment, the pods are substantially egg shaped.

Pods may be made from any suitable material. In one embodiment, the pods may comprise a cellulose matrix which allows the leaching of drugs contained within the matrix into the fluids of the body cavity.

The pods may be attached to the support frame by a variety of means.

For example, there may be a complementary plug and socket between the pod and the support frame allowing the pod to be readily attached to and subsequently detached from the frame. This would enable reloading of the device to prolong a treatment. The design would also enable concurrent treatments of different drugs or substances to be applied from two or more pods through different stages of a treatment cycle by removing the device and placing new pods for immediate reinsertion thereby creating no disruption to the current treatment cycle and similarly the same treatment may be prolonged by replacing pods.

Thus, the present invention can provide two or more co-current treatments, two or more sequential treatments or prolong a single treatment. All of which are achievable by the ability to replace pods.

In another embodiment the pod will be configured so as to have a portion of the pod slide into a groove on the support frame (or vice versa).

Other attachment mechanisms may be the mating of uneven surfaces (such as in Velcro^{™}).

Another method may the use of a suitable adhesive.

However, in preferred embodiments the pod is flexibly attached to the support frame allowing full movement of the pod with respect to the support frame. This enables the surfaces of the pod to move gently against the lining of the body cavity (or not at all) even if there is a violent movement of the support frame holding the pods.

It should be appreciated that if the pods have a curved surface as previously described, and the arms are tensioned gently outwards, the flexible attachment allows the surface of the pod to gently contact the mucosal membrane of the vagina without irritation allowing ready transfer of the drugs contained within the pods. It should be noted that some treatments will be enhanced by mucosal membrane contact whereas other treatments can be transmitted effectively through delivery into the vaginal mucosa and fluids.

It is envisaged that there are many ways by which the flexible attachment may be achieved.

In one embodiment this is by a ball and socket arrangement allowing three dimensional movement of the pod with respect to the support frame.

It should be seen that the tensioning of the arms outwards enables the device to be retained in the body cavity when *in situ.* However, the use of pliable arms means that the arms can be moved to allow the device to be effectively compressed to allow ready insertion and withdrawal of the device through the orifice to the body cavity.

In one embodiment, the device is capable of having its arms wrapped around itself or merely compressed together.

In another embodiment to the present invention the arms are capable of interlocking for removal or insertion.

For example, the main body of the arms may be designed such that the stem is made in two adjacent webs that are joined by connecting braces at regular intervals. The adjacent arms of the device facing one another may be slightly offset. This enables the arms to be forced together so the upper arch of wishbones on adjacent webs intertwine to enable the adjacent pods to close together to the narrowest position.

If webs are used, then the device has less material giving a lighter frame and therefore is less likely to cause adverse tissue reactions.

It should be appreciated that the pods can be positioned anywhere in relative to the support frame. However, preferred embodiments of pods are attached at or near the distal end of the arms of the support frame. In some embodiments there may be more than one pod on an arm.

In preferred embodiments of the present invention there is provided a locator to enable the device *in situ* to be readily located and removed from the animal. This locator in some instances may be an aperture.

It can be seen that the present invention has considerable advantages over the

### prior art.

Usage of attachable pods enable the support frame of the device to be readily reused which leads to economical savings.

Further, only the pods need to be disposed of giving environmental advantages. Furthermore, the pods can be assessed for residual drug containment and if necessary be disposed according to environmental safety requirements if treatment has not depleted the drug.

The ability to remove pods means that treatment of the animal or human can be changed in the treatment through the removal of the device and the substitution of a pod or more.

Treatments can also be customised with different pods use, perhaps containing different drugs or different dosage rates.

The flexible attachment of the pods to the support frame means that the pods are free moving and able to orientate themselves in accordance with mucosal membrane movement and device orientation. This enables the pods to provide interface with the mucosal membrane in some instances enhance the delivery and transmission of drugs and nutrients.

The wishbone configuration of preferred embodiments provides a gentle tensioning of the arms in comparison with rigid devices used previously.

Manufacture of the support frame is considerably easier than previously as there is no need to consider the impregnation of drugs into material from which the support frame is manufactured.

Finally, the present invention allows for ready insertion and removal.

Aspects of the present invention will now be described by way of example only with reference to the accompanying drawings in which:
- Figure 1: diagrammatic view of substance delivery device in accordance with one embodiment on the present invention, and
- Figure 2: diagrammatic drawing of the device in figure 1 in an insertion/removal configuration.

With respect to the figures, there is illustrated a drug system delivery device generally indicated by arrow 1.

The device 1 includes a support frame 2 attached to which are substance delivery pods 3.

The support frame 2 is in the form of a wishbone having two arms 4 and 5 substantially S-shaped connected to an elongated base 6.

The base 6 contains a locator, in the form of an aperture 8, for easy location and removal of the device. Note the base is not associated with any flexing which only occurs along the S-shaped arms.

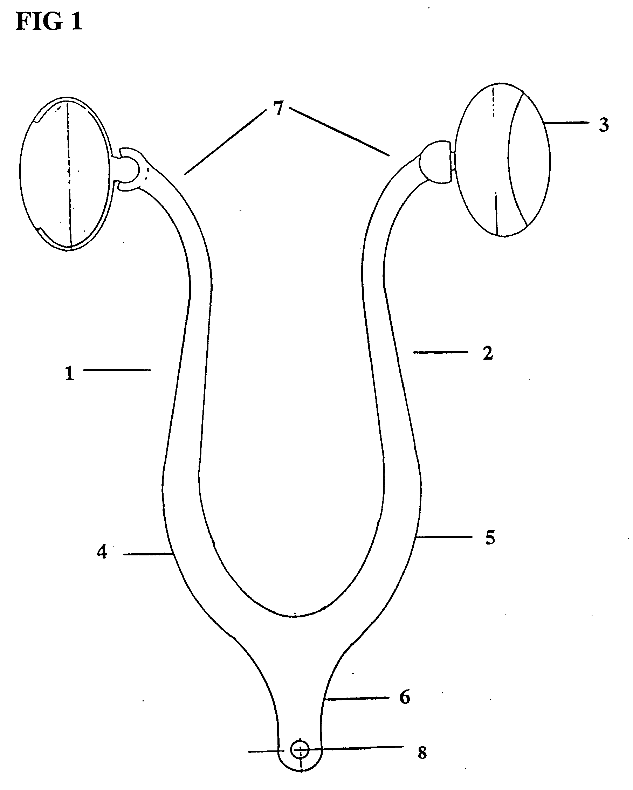

The arms 4 and 5 are curved in such a manner that when *in situ* (refer Figure 1) the distal ends of the arms 7 are biased outwards.

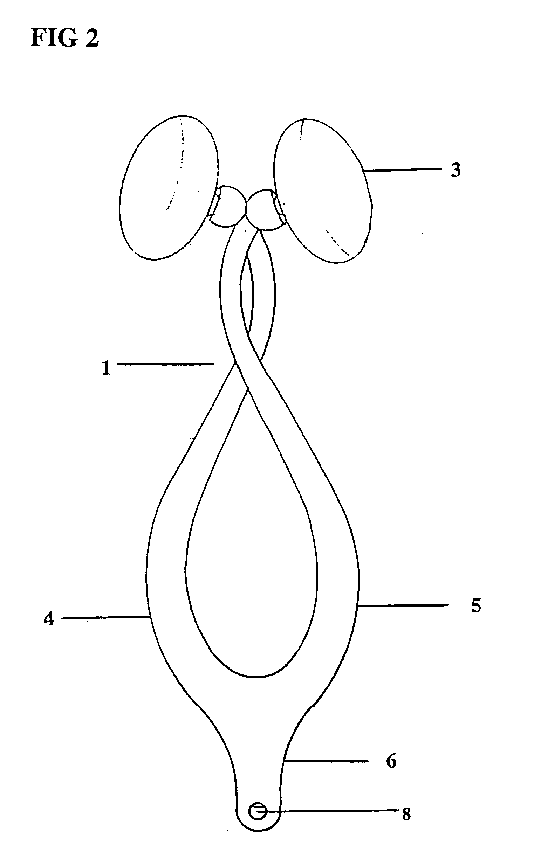

It is envisaged that the configuration of the arms and the flexibility of the material from which the support frame will be made will enable the arms to move in such a fashion so as to cross-over as illustrated in Figure 2. There may be provided webbing (not shown) to interlock the arms. This cross-over configuration allows for ready insertion and removal of the device.

The pods 3 are housings which contain a drug delivered into the body cavity. In this embodiment, housing of pods 3 is cellulose or an appropriate matrix.

The pods 3 are attached to the arms 4 and 5 by a flexible attachment in the form of a ball and socket (not clearly shown).

It can be seen that the curved outer shape of the pods 3 in combination with the biasing of the arms 4 and 5 and the flexible attachment allows free movement of the pods against the mucosal membrane without irritating the membrane.

Aspects of the present invention are described by way of example only and it should be appreciated that modifications and additions may be made thereto without departing from the scope of the appended claims.

## Claims

1. An internal substance delivery device (1) for insertion into a body cavity, said device including a support frame (2) and a substance delivery means (3) which is capable of releasing substance into the body cavity, the support frame having at least two resilient arms (4, 5) for retaining said device in the body cavity, **characterised in that** each resilient arm is arranged to receive and to release said substance delivery means (3).

2. A substance delivery device as claimed in claim 1, wherein the substance delivery means is loaded with a drug.

3. A substance delivery device (1) as claimed in claim 1 or claim 2, wherein the said device is an intra-vaginal release device.

4. A substance delivery device (1) as claimed in any preceding claim, wherein the substance delivery means (3) is arranged to release the substance through osmosis.

5. A substance delivery device (1) as claimed in any preceding claim, wherein the substance delivery means (3) are rounded.

6. A substance delivery device (1) as claimed in any preceding claim, wherein the substance delivery means (3) is flexibly attached to the arms (4, 5).

7. A substance delivery device (1) as claimed in any preceding claim, wherein the substance delivery means (3) is attached to the arms (4, 5) by respective ball and socket mechanisms.

8. A substance delivery device (1) as claimed in any preceding claim, wherein the support frame (2) is in the form of a wish bone.

9. A substance delivery device (1) as claimed in any preceding claim, wherein the arms (4, 5) are biased outward from a central section of the support frame (2).

10. A substance delivery device (1) as claimed in any preceding claim, wherein the support frame (2) is made of nylon.

11. A substance delivery device (1) as claimed in any preceding claim, wherein the arms (4, 5) are sufficiently pliable to be moved together to allow the substance delivery device to be effectively compressed.

12. A substance delivery device (1) as claimed in any preceding claim, wherein the arms (4, 5) are capable of interlocking for removal or insertion.

13. A substance delivery device (1) as claimed in any preceding claim, wherein the support frame (2) includes a locator (8) to enable the substance delivery device (1) to be readily located and removed from *in situ.*

## Patentansprüche

1. Interne Substanzabgabevorrichtung (1) zum Einsetzen in eine Körperhöhle, wobei die Vorrichtung eine Halterahmen (2) und ein Substanzabgabemittel (3) umfasst, das in der Lage ist, eine Substanz in die Körperhöhle freizusetzen, wobei der Halterahmen mindestens zwei elastische Arme (4, 5) zum Halten der Vorrichtung in der Körperhöhle besitzt, **dadurch gekennzeichnet, dass** jeder elastische Arm so ausgebildet ist, dass er das genannte Substanzabgabemittel (3) aufnehmen und freigeben kann.

2. Substanzabgabevorrichtung wie in Anspruch 2 beansprucht, worin das Substanzabgabemittel mit einem Medikament beladen ist.

3. Substanzabgabevorrichtung (1) wie in Anspruch 1 oder Anspruch 2 beansprucht, worin die genannte Vorrichtung eine intravaginale Freisetzungsvorrichtung ist.

4. Substanzabgabevorrichtung (1) wie in einem der voranstehenden Ansprüche beansprucht, worin das Substanzabgabemittel (3) so ausgebildet ist, dass die Substanz durch Osmose freigesetzt wird.

5. Substanzabgabevorrichtung (1) wie in einem der voranstehenden Ansprüche beansprucht, worin das oder die Substanzabgabemittel (3) gerundet ist/sind.

6. Substanzabgabevorrichtung (1) wie in einem der voranstehenden Ansprüche beansprucht, worin das Substanzabgabemittel (3) biegsam oder elastisch an den Armen (4, 5) befestigt ist.

7. Substanzabgabevorrichtung (1) wie in einem der voranstehenden Ansprüche beansprucht, worin das Substanzabgabemittel (3) mit Hilfe entsprechender Kugelgelenk-Einrichtungen an den Armen (4, 5) befestigt ist.

8. Substanzabgabevorrichtung (1) wie in einem der voranstehenden Ansprüche beansprucht, worin der Halterahmen (2) die Gestalt eines Schwingarms oder einer Wünschelrute besitzt.

9. Substanzabgabevorrichtung (1) wie in einem der voranstehenden Ansprüche beansprucht, worin die Arme (4, 5) von einem Mittelabschnitt des Halterahmens (2) ausgehend nach außen gespannt sind.

10. Substanzabgabevorrichtung (1) wie in einem der voranstehenden Ansprüche beansprucht, worin der Halterahmen (2) aus Nylon hergestellt ist.

11. Substanzabgabevorrichtung (1) wie in einem der voranstehenden Ansprüche beansprucht, worin die Arme (4, 5) ausreichend biegsam sind, um gegeneinander bewegt zu werden, damit die Substanzabgabevorrichtung wirksam zusammengedrückt werden kann.

12. Substanzabgabevorrichtung (1) wie in einem der voranstehenden Ansprüche beansprucht, worin die Arme (4, 5) in der Lage sind, zum Zwecke des Entfernens oder Einsetzens gekoppelt oder geklammert zu werden.

13. Substanzabgabevorrichtung (1) wie in einem der voranstehenden Ansprüche beansprucht, worin der Halterahmen (2) eine Positionierhilfe (8) umfasst, um sicherzustellen, dass die Substanzabgabevorrichtung (1) leicht an ihren Ort verbracht und von dort entfernt werden kann.

## Revendications

1. Dispositif interne d'administration d'une substance (1) destiné à être inséré dans une cavité corporelle, ledit dispositif comprenant un cadre support (2) et un moyen d'administration d'une substance (3) qui est capable de libérer une substance dans la cavité corporelle, le cadre support possédant au moins deux bras élastiques (4, 5) pour maintenir ledit dispositif dans la cavité corporelle, **caractérisé en ce que** chaque bras élastique est disposé pour recevoir et libérer ledit moyen d'administration d'une substance (3).

2. Dispositif d'administration d'une substance selon la revendication 1, dans lequel le moyen d'administration d'une substance est chargé d'un médicament.

3. Dispositif d'administration d'une substance (1) selon la revendication 1 ou la revendication 2, dans lequel ledit dispositif est un dispositif d'administration intravaginale.

4. Dispositif d'administration d'une substance (1) selon l'une quelconque des revendications précédentes, dans lequel le moyen d'administration d'une substance (3) est disposé pour libérer la substance par osmose.

5. Dispositif d'administration d'une substance (1) selon l'une quelconque des revendications précédentes, dans lequel les moyens d'administration d'une substance (3) sont arrondis.

6. Dispositif d'administration d'une substance (1) selon l'une quelconque des revendications précédentes, dans lequel le moyen d'administration d'une substance (3) est fixé de façon flexible aux bras (4, 5).

7. Dispositif d'administration d'une substance (1) selon l'une quelconque des revendications précédentes, dans lequel le moyen d'administration d'une substance (3) est fixé aux bras (4, 5) par des mécanismes à rotule correspondants.

8. Dispositif d'administration d'une substance (1) selon l'une quelconque des revendications précédentes, dans lequel le cadre support (2) a la forme d'une fourche.

9. Dispositif d'administration d'une substance (1) selon l'une quelconque des revendications précédentes, dans lequel les bras (4, 5) dévient vers l'extérieur à partir d'une section centrale du cadre support (2).

10. Dispositif d'administration d'une substance (1) selon l'une quelconque des revendications précédentes, dans lequel le cadre support (2) est en nylon.

11. Dispositif d'administration d'une substance (1) selon l'une quelconque des revendications précédentes, dans lequel les bras (4, 5) sont suffisamment souples pour être déplacés ensemble afin de permettre que le dispositif d'administration d'une substance soit efficacement comprimé.

12. Dispositif d'administration d'une substance (1) selon l'une quelconque des revendications précédentes, dans lequel les bras (4, 5) sont capables de s'entrecroiser pour le retrait ou l'insertion.

13. Dispositif d'administration d'une substance (1) selon l'une quelconque des revendications précédentes, dans lequel le cadre support (2) comprend un repère (8) pour permettre que le dispositif d'administration d'une substance (1) soit aisément localisé et retiré de sa position in situ.
